# EUROPEAN PATENT SPECIFICATION

(11) **EP 2 398 882 B1**
(45) Date of publication and mention of the grant of the patent: **13.07.2016**
(21) Application number: 10705449.6
(22) Date of filing: 16.02.2010
(51) Int. Cl.: C11B 1/02, C11B 1/04, B04B 1/20

(54) **A CONTINUOUS PROCESS FOR ISOLATION OF OILS FROM ALGAE OR MICRO ORGANISMS**
KONTINUIERLICHES VERFAHREN ZUR ISOLIERUNG VON ÖLEN AUS ALGEN ODER MIKROORGANISMEN
PROCÉDÉ CONTINU POUR ISOLER DES HUILES À PARTIR D'ALGUES OU DE MICRO-ORGANISMES

(30) Priority: 17.02.2009 SE 0950085
(43) Date of publication of application: 28.12.2011
(73) Proprietor: Alfa Laval Corporate AB, 221 00 Lund (SE)
(72) Inventor: WASE, Claes, S-146 38 Tullinge (SE); RIDDERSTRÅLE, Rolf, S-117 31 Stockholm (SE)
(74) Representative: Alfa Laval Attorneys
(86) International application number: PCT/SE2010/050176
(87) International publication number: WO 2010/096002

(56) References cited:
- WO-A1-99/65610
- WO-A1-2008/151373
- US-A- 5 229 014
- US-A1- 2004 229 325
- US-A1- 2008 090 284
- US-A1- 2008 160 593

## Description

The present invention relates to a process for isolation of oils from algae or micro organisms, and use of the process for production of bio-diesel or bio-fuels.

US 2004/0229325 discloses a process for lipid extraction from microorganisms and comprises a step of lysing cells of the microorganisms and separating heavy from light phases to obtain the lipid in the light phase. Such separation may be performed by centrifugation.

WO 2008/151373 discloses a method and system for algae growth for making bio fuels. When harvesting algae, these may be separated from water using a parallel plate separator. For extracting the oil from the algae, a wet extraction process is used in which extractant is added to an algae concentrate. The oil/extractant mixture is then separated from the remaining biomass and water either using gravity settling or centrifugation.

### The Invention

The majority of algae that are intentionally cultivated fall into the category of micro-algae, they are also referred to as phytoplankton, micro-phytes, or planktonic algae. Macro-algae, commonly known as seaweed, have many commercial and industrial uses, but due to their size and the specific requirements of the environment in which they need to grow, they do not lend themselves as readily to cultivation, but still they are of interest for the present invention. Commercial and industrial purposes of algae cultivation are for production of bio-plastics, dyes, colorants, feedstock, pharmaceuticals, pollution control, algae fuel which could be converted to biodiesel and to bio fuels. Thus, lots of substances can be isolated from algae and there is lots of commercial interest in developing cost effective processes for the different purposes.

There are several steps in the bio-diesel or bio-fuel production processes where centrifugation is useful. The algae, according to one alternative of the invention, may be separated from the algae slurry. Oils may be extracted from the algae before the oils are separated in a second centrifugation step and before any reaction to produce bio-diesel can be accomplished. Separation of bio-diesel etc. together with any leftover reactants may be removed by centrifugation. The bio-diesel may be further treated and centrifuged.

Trans-esterification of algal oil is normally done with ethanol and sodium ethanolate serving as the catalyst. Sodium ethanolate can be produced by reacting ethanol with sodium. Thus, with sodium ethanolate as the catalyst, ethanol is reacted with the algal oil to produce bio-diesel and glycerol. This end-mixture may be separated by the use of a centrifuge.

One problem with isolation of oils from an algae-slurry is the huge quantities of water in the slurry. Another problem is how to extract the oils from the algae cells. A third problem is how to economize such a process.

The problems are solved according to the present invention by use of centrifugal separators. According to the invention, a two phase and a three phase centrifugal separators are used to recover the algae oils. The present invention thus relates to a process for isolation of the oils from the algae slurry, which process comprises three process steps, the last step being carried out in a three phase centrifugal separator. According to this aspect of the present invention the problem is solved by a continuous process for isolation of oils from an algae slurry or a micro organism slurry, which process comprises concentration of the slurry as described later on, liberation of oils by rupturing or permeabilisation of cell walls of the micro organisms or cell walls of the algae, feeding the slurry into a three phase centrifugal separator having a stack of separating discs, and which three phase centrifugal separator is operating under a force of at least 4000 G, preferably under a force of at least 4500 G, most preferred under a force of at least 5000 G. Any type of three phase centrifugal separators can be used as long as the three phase separator has a stack of separating discs and is operating under force of at least 4000 G, preferably under a force of at least 4500 G, most preferred under a force of at least 5000 G. By exposing the slurry to the centrifugal force three phases are obtained. The three phases comprises one oil phase, one liquid phase and one bio-organic phase, wherein the outgoing bio-organic phase has a dryness of at least 30%, preferably a dryness of at least 35%, most preferred a dryness of at least 50%, and wherein the bio-organic phase contains cell parts. The rupture of the cell walls could be carried out by friction caused by the centrifugal force, the high speed, and the contact with the centrifugal bowl wall. The ruptures could be enhanced by additional operations such as ultra sound, heat or by any other suitable method. Another possibility could be grounding of the cells or rupturing or permeabilisation of the cell walls of the micro organisms or the cell walls of the algae in a step before the separation in the three phase centrifugal separator, the rupturing or the permeabilisation can suitably be made by one or more methods within the group consisting of ultra sonication, liquid shear disruption, bead milling, high pressure pressing, freeze-thawing, freeze-pressing, enzymatic digestion, hydrolysation, and virus degradation.

The continuous process according to the invention comprise conveying the bio-organic phase out of the three phase separator by a conveyor screw. The conveyor screw could comprise a central core, which extends axially through the whole of the lower rotor portion of the separator, a sleeve-formed part comprises a number of apertures, which are distributed round the axis of rotation R and extend axially from the upper portion of the screw conveyor down in a screw-like manner along the whole inside of the rotor body from the latter's upper end to its lower end. The conveyor screw outlet for the bio-organic is here named first outlet.

Two paring discs are paring out the oil phase and the liquid phase, thus one paring discs could be paring out the oil phase and one paring disc could be paring out the liquid phase according to the present invention. At the upper end of a rotor body is at least one outlet for fluids. An outlet channel for the purified liquid, the oils, extends in an outlet pipe which surrounds the inlet pipe for the feed of algae and micro organism slurry, and defines the second outlet. The second outlet may constitute a space for collecting of fluids and a paring disc for discharge of fluids from this space.

The upper end of the rotor body could be provided with an outlet for relatively higher density fluids defined as the third outlet. This outlet could be configured in substantially the same way as the second outlet for relatively lower density fluids. Thus a further space in the form of an outlet chamber for higher density fluids could be formed between the conveyor shaft and the outlet chamber for lower density fluids. A paring disc for discharge of higher density fluids is arranged within this outlet chamber, wherein the paring disc communicates with an outlet channel for fluids.

The conveyor shaft could comprise a number of holes, which connect an annular space situated radially outside the stack of separation discs with the outlet chamber for higher density fluids. The holes could be adapted to form an overflow outlet corresponding to the outlet for fluids in the rotor body which flow towards and out through the outlet for higher density fluids, in such a way that an interface level between higher density fluids and lower density fluids could be maintained at a radial level in the rotor body.

In the continuous process can the outgoing bio-organic phase have a dryness of at least 30%, preferably a dryness of at least 35%, most preferred a dryness of at least 50%. The bio-organic phase contains cell parts such as cell walls and other cell materials which constitute the original cell.

The algae or the micro organisms could be dried before the oil content could be pressed out with an oil press. Since different strains of algae vary widely in their physical attributes, various press configurations screws, expellers, pistons, etc work better for specific algae types. Many commercial manufacturers of vegetable oil use a combination of mechanical pressing and chemical solvents in extracting oils.

The rupturing or permeabilisation of the cell walls of the micro organisms or the cell walls of the algae can suitably be made by one or more methods within the group consisting of ultra sonication, liquid shear disruption, bead milling, high pressure pressing, freeze-thawing, freeze-pressing, enzymatic digestion, hydrolysation, and virus degradation. The rupturing of the cell walls and thus liberation of oils can be done within the rotor body continuously at the periphery of the separator, but the process may also comprise an extra step for liberation of oils by any one of the methods before feeding the slurry into the three phase centrifugal separator. According to one alternative could the slurry pass an ultra sound device before entering the three phase centrifugal separator.

Ultrasonic extraction may greatly accelerate extraction processes. Using an ultrasonic reactor, ultrasonic waves could be used to create cavitations bubbles in a solvent material, when these bubbles collapse near the cell walls, can they create shock waves and liquid jets that cause those cell walls to break and release their contents into the solvent.

According to the present invention the process for isolation of oils begins with a concentration step wherein the slurry is concentrated in a two phase separator having a stack of separating discs and is operating under force of at least 4000 G, preferably under a force of at least 4500 G, most preferably 5000 G.

The present invention relates to a continuous process, which process comprises three steps, which in an embodiment comprises:

### Step 1:

Feeding micro organism slurry or algae slurry into a two phase centrifugal separator having a stack of separating discs and operating under force of at least 4000 G, preferably under a force of at least 4500 G, most preferably 5000 G, obtaining a cell phase having a dryness of at least 15%, preferably of at least 18%, most preferred at least 20%.

### Step 2:

Liberation of oils by rupturing or permeabilisation of cell walls of the cells in the cell phase by one or more methods within the group consisting of ultra sonication, liquid shear disruption, bead milling, high pressure pressing, freeze-thawing, freeze-pressing, enzymatic digestion, hydrolysation, and virus degradation, obtaining a slurry of liquids, oil and cell parts.

### Step 3:

Feeding the slurry from step 2 into a three phase centrifugal separator having a stack of separating discs and operating under force of at least 4000 G, preferably under a force of at least 4500 G, most preferably 5000 G, obtaining three phases: one oil phase, one liquid phase and one bio-organic phase containing cell parts.

In the three step continuous process according to the invention the cell phase obtained in step 1 is conveyed out of the two phase centrifugal separator by a conveyor screw. The bio-organic phase containing cell parts from step 3 is also conveyed out of the three phase centrifugal separator by a conveyor screw.

In the continuous process according to the invention could the liquid phase and/or the bio-organic phase containing the cell parts be further processed to obtain cellulose and/or starch, which could be further processed to obtain methanol, ethanol, methanol derivatives or ethanol derivatives. These further process products could constitute a base for the production of biodiesel or bio-fuels from the separated oils.

The oil may be extracted by a solvent such as methanol, ethanol, ethyl acetate or any other suitable solvent.

The continuous process of the invention is used for production of biodiesel or bio fuels.

Three phase and two phase centrifugal separators, which are used according to the present invented process, are explained more closely by a description of various embodiments of the separators and with reference to the drawings attached hereto.

### Brief description of the figures

- Figure 1: discloses detailed view of a centrifugal separator according to an embodiment.
- Figure 2: discloses a detailed view of a centrifugal separator according to a further embodiment.
- Figure 3: discloses a detailed view of a centrifugal separator according to a further embodiment.

### Detailed description of the figures

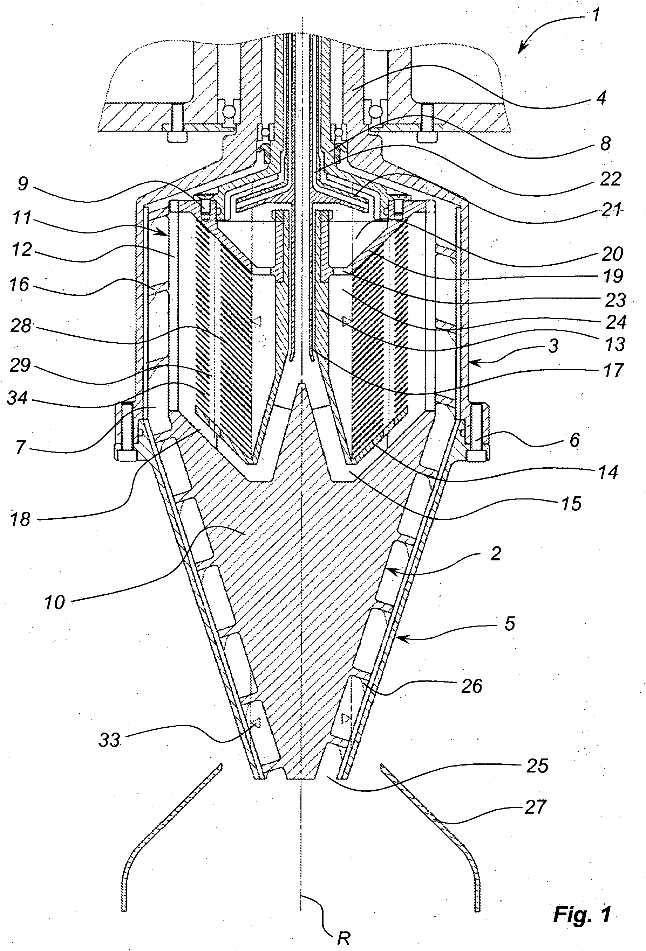

Figure 1 discloses an example of a centrifugal separator comprising a rotor body 1 which is rotatable at a certain speed about a vertical axis of rotation R, and a screw conveyor 2 which is arranged in the rotor body 1 and rotatable about the same axis of rotation R but at a speed which differs from the rotation speed of the rotor body 1.

The centrifugal separator is intended to be suspended vertically in a manner indicated by WO 99/65610. The device necessary for suspending and driving the centrifugal separator is therefore not described here.

The rotor body 1 has an essentially cylindrical upper rotor portion 3 comprising or connected to a hollow rotor shaft 4, and an essentially conical lower rotor portion 5. The rotor portions 3 and 5 are connected to one another by screws 6 and delimit a separation chamber 7. Alternative connecting organs may of course be used.

A further hollow shaft 8 extends into the rotor body 1 via the inside of the rotor shaft. The shaft 8 bears the screw conveyor 2 and they are connected to one another by screws 9. The hollow shaft 8 is drivingly connected to the screw conveyor 2.and is hereinafter called the conveyor shaft.

As illustrated in Figure 1, the screw conveyor 2 comprises a central core 10, which extends axially through the whole of the lower rotor portion, a sleeve-formed part 11 comprising a number of apertures 12 which are distributed round the axis of rotation R and extend axially from the upper portion of the screw conveyor 2 to the conical portion of the screw conveyor 2, a number of wings 15 which are distributed round the axis of rotation R and connect the core 10 to a central sleeve 13 situated at a radial distance from the axis of rotation R within the sleeve-formed part 11 of the screw conveyor 2, which central sleeve 13 changes to a conical portion and a lower support plate 14, and at least one conveying thread 16 which extends in a screw-like manner along the whole inside of the rotor body 1 from the latter's upper end to its lower end and is itself connected to the sleeve-formed part 11 and the core 10. The at least one conveying thread 16 may of course be supplemented by a suitable number of conveying threads, e.g. two, three or four, which all extend in a screw-like manner along the inside of the rotor body 1.

An inlet pipe 17 for feeding liquid mixtures which are to be treated in the rotor body 1 extends through the conveyor shaft 8 and leads on into the central sleeve 13. The inlet pipe 17 discharges axially before said wings 15 into a space centrally in the screw conveyor 2. Axially closer to the core 10, the core and the lower support plate 14 form a passage 18 which constitutes a continuation of the inlet channel which extends through the inlet pipe 17. The passage 18 is in communication with the inside of the rotor body 1 via channels between the wings 15.

A space in the form of an outlet chamber 20 is formed between the conveyor shaft 8 and an upper conical support plate 19. A paring disc 21 for discharging purified liquid is disposed within the outlet chamber 20. The paring disc 21 is firmly connected to the inlet pipe 17. An outlet channel 22 for the purified liquid extends in an outlet pipe which surrounds the inlet pipe 17 and defines the second outlet.

A centrally and axially directed outlet 25 for separated dry phase 26 is arranged at the lower end of the rotor body 1 and defines the first outlet. In connection with this outlet 25 for dry phase 26, the rotor body 1 is surrounded by a device 27 for intercepting dry phase 26 which leaves the outlet 25. The dry phase 26 is disclosed in the drawings in the form of accumulations at the radially outer portion of the conveying thread 16, on the latter's side which faces towards the first outlet 25.

The rotor body 1 further comprises a stack of truncated conical separation discs 28 which are examples of surface-enlarging inserts. These are fitted coaxially with the rotor body 1 centrally in its cylindrical portion 3. The conical separation discs 28, which have their base ends facing away from the outlet 25 for the separated dry phase, are held together axially between the upper conical support plate 19 and the lower conical support plate 14 by the central sleeve 13 which extends through the stack of truncated conical separating discs 28. The separation discs 28 comprise holes which form channels 29 for axial flow of liquids when the separation discs 28 are fitted in the centrifugal separator. The upper conical support plate 19 comprises a number of apertures 23 which connect the space 24 situated radially within the stack of separation discs to the outlet chamber 20.

Alternatively, the conical separation discs 28 may be so oriented that they have their base ends facing towards the outlet 25 for separated dry phase.

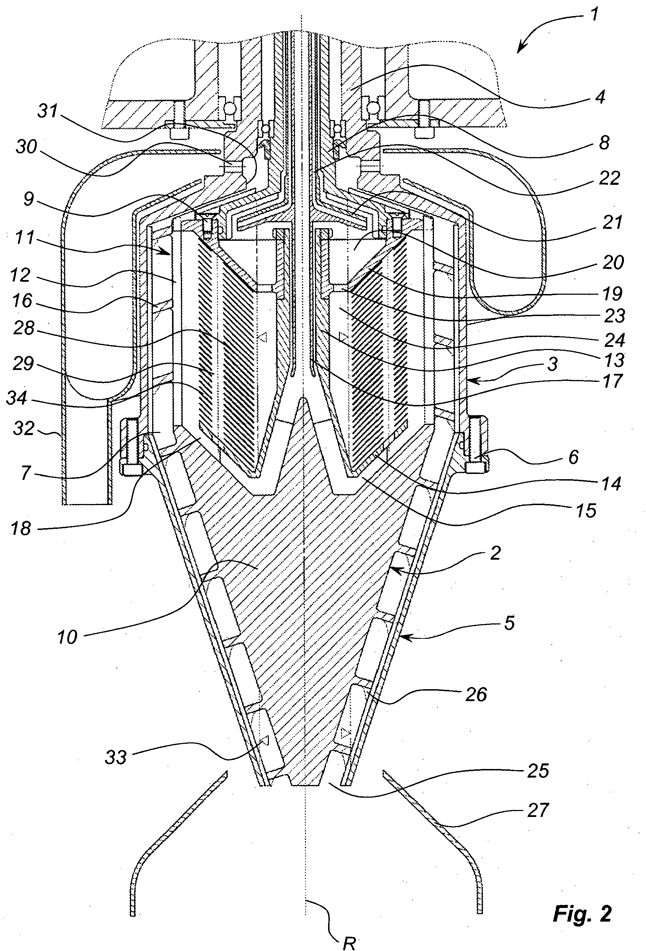

The parts in Figure 1 which are the same have corresponding reference signs in Figure 2.

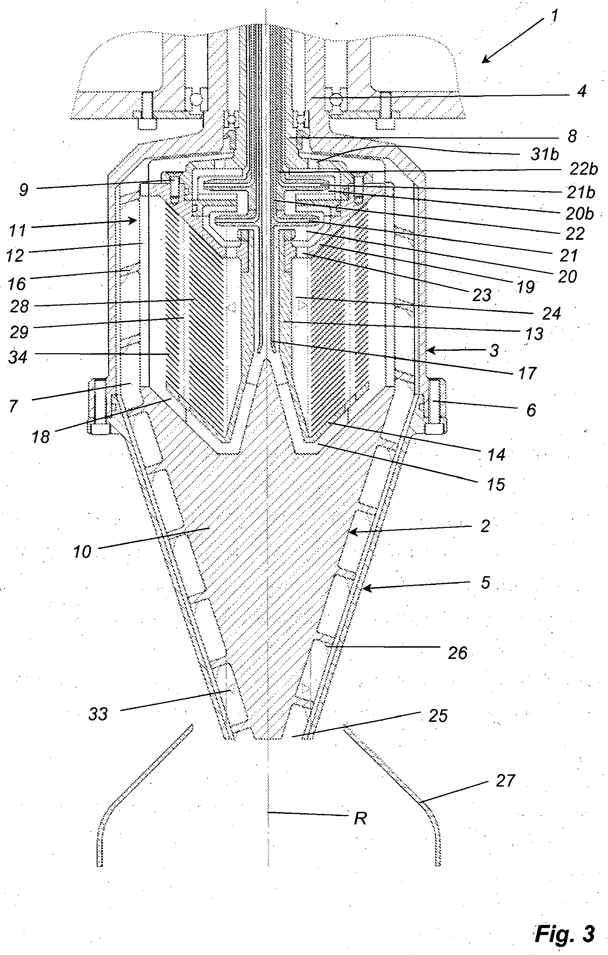

Figure 2 discloses a further embodiment of the centrifugal separator in which the rotor body 1 at its upper end comprises at least one outlet 30 for fluids with a higher density than the fluids which have been purified and is led out through said paring disc 21, which at least one outlet 30 defines the third outlet. In the region of the at least one outlet 30, somewhat below this outlet, a flange is arranged which forms an overflow outlet 31 for fluids in the rotor body 1 which flows towards and out through the at least one outlet 30. The flange's overflow outlet 31 is adapted to maintaining an interface level between higher density fluids and lower density fluids in the rotor body 1 at a radial level (level not disclosed in the figure). This interface level can be regulated radially in the separation chamber 7 by selecting the extent of the overflow outlet 31 in the radial direction. According to the embodiment disclosed in Figure 2, the centrifugal separator comprises a device 32 which surrounds the rotor body 1 and is adapted to intercepting liquid which leaves the rotor body 1 through the at least one outlet 30. Figure 2 discloses the at least one outlet 30 as an open outlet. Alternatively, this outlet may also, in the same way as at the second outlet 22, be provided with a space for collecting of fluids and a paring disc for discharge of fluids from this space. Such an alternative outlet - to the open outlet disclosed in Figure 2 - is disclosed in Figure 3. The parts in Figure 2 which are the same have corresponding reference signs in Figure 3.

Figure 3 discloses accordingly a further embodiment of the centrifugal separator provided with said alternative outlet for relatively higher density fluids. To this end, the outlet is configured in substantially the same way as the second outlet 22 for relatively lower density fluids. Thus a further space in the form of an outlet chamber 20b for higher density fluids is formed between the conveyor shaft 8 and the outlet chamber 20 for lower density fluids (purified liquid). A paring disc 21b for discharge of higher density fluids is arranged within this outlet chamber 20b, wherein the paring disc 21 b communicates with an outlet channel 22b for fluids. The outlet channel 22b for higher density fluids extends in an outlet pipe which surrounds the outlet pipe and the outlet channel 22 for lower density fluids (purified liquid). The conveyor shaft 8 comprises a number of holes 31b which connect an annular space situated radially outside the stack of separation discs with the outlet chamber 20b for higher density fluids. The holes 31 b are adapted to form an overflow outlet corresponding to that disclosed in Figure 2 for fluids in the rotor body 1 which flow towards and out through the outlet for higher density fluids, in such a way that an interface level between higher density fluids and lower density fluids is maintained at a radial level (level not disclosed in Figure 3) in the rotor body 1. The outlet described with the paring disc makes it possible for the centrifugal separator's outlet 22b for higher density fluids to be adapted, instead of communicating with said device 32 (in Figure 2) which surrounds the rotor body in order to intercept liquid which leaves the open outlet, to communicate with a collection device (such as a collection tank) which may be arranged at a distance from, and at a higher level than, the centrifugal separator (not disclosed in Figure 3). Fluids are thus pumped out from the centrifugal separator to the collection device through the paring disc.

## Claims

1. A continuous process for isolation of oils from a micro organism slurry or an algae slurry comprising concentration of the slurry of micro organisms or the slurry of algae in a two phase centrifugal separator having a stack of separating discs and is operating under force of at least 4000 G, preferably under a force of at least 4500 G, most preferably 5000 G, obtaining a cell phase and conveying out the cell phase from the two phase centrifugal separator by conveyor screw (2);
liberation of oils by rupturing or permeabilisation of cell walls of the cells in the obtained cell phase obtaining a slurry comprising liberated oils and ruptured cell walls;
separating the slurry comprising liberated oils and ruptured cell walls in a three phase centrifugal separator having a stack of separating discs and operating under force of at least 4000 G, preferably under a force of at least 4500 G, most preferably 5000 G, obtaining three phases: one oil phase, one liquid phase and one bio-organic phase containing cell parts, wherein the bio-organic phase containing cell parts from is conveyed by a out of the three phase centrifugal separator by conveyor screw (2), and wherein two paring discs are paring out the oil phase and the liquid phase, one paring discs is paring out the oil phase and one paring disc is paring out the liquid phase.

2. The continuous process according to claim 1, wherein the liberation of oils by rupturing or permeabilisation of cell walls of the micro organisms or cell walls of the algae is carried out by one or more methods within the group consisting of sonication, liquid shear disruption, bead milling, high pressure pressing, freeze-thawing, freeze-pressing, enzymatic digestion, hydrolysation, and virus degradation.

3. The continuous process according to claim 1 or 2, wherein a cell phase is obtained from the two phase separator, which cell phase has a dryness of at least 15 %, preferably of at least 18%, most preferably of at least 20%.

4. The continuous process according to any one of the preceding claims, wherein the outgoing bio-organic phase has a dryness of at least 30%, preferably a dryness of at least 35%, most preferred a dryness of at least 50%.

5. The continuous process according to any one of the preceding claims, wherein one paring disc is paring out the liquid phase from the two phase separator.

6. The continuous process according to any one of the preceding claims, wherein the liquid phase and/or the bio-organic phase containing the cell parts are further processed to obtain cellulose and/or starch, which could be further processed to obtain alcohol, such as methanol or ethanol, or alcohol derivatives.

7. Use of a process according to any one of claims 1 to 6 for production of biodiesel or bio fuels.

## Patentansprüche

1. Kontinuierliches Verfahren zur Isolierung von Ölen aus einer Mikroorganismenaufschlämmung oder einer Algenaufschlämmung, umfassend das Konzentrieren der Aufschlämmung von Mikroorganismen oder der Aufschlämmung von Algen in einem Zweiphasen-Zentrifugentrenner, der einen Stapel von Trennscheiben aufweist und unter einer Kraft von mindestens 4000 G, bevorzugt unter einer Kraft von mindestens 4500 G, am bevorzugtesten von 5000 G betrieben wird, wobei eine Zellphase erhalten wird, und das Fördern der Zellphase aus dem Zweiphasen-Zentrifugentrenner durch eine Förderschnecke (2);
das Freisetzen von Ölen durch Aufbrechen oder Permeabilisierung von Zellwänden der Zellen in der erhaltenen Zellphase, wobei eine Aufschlämmung erhalten wird, die freigesetzte Öle und aufgebrochene Zellwände umfasst;
das Trennen der die freigesetzten Öle und aufgebrochenen Zellwände umfassenden Aufschlämmung in einem Dreiphasen-Zentrifugentrenner, der einen Stapel von Trennscheiben aufweist und unter einer Kraft von mindestens 4000 G, bevorzugt unter einer Kraft von mindestens 4500 G, am bevorzugtesten von 5000 G betrieben wird, wobei drei Phasen erhalten werden: eine Ölphase, eine flüssige Phase und eine bioorganische Phase, die Zellteile enthält, wobei die bioorganische Phase, die Zellteile enthält, aus dem Dreiphasen-Zentrifugentrenner durch die Förderschnecke (2) befördert wird und wobei zwei Schälscheiben die Ölphase und die flüssige Phase auseinander schälen, wobei eine Schälscheibe die Ölphase ausschält und eine Schälscheibe die flüssige Phase ausschält.

2. Kontinuierliches Verfahren nach Anspruch 1, wobei die Freisetzung von Ölen durch Aufbrechen oder Permeabilisierung von Zellwänden der Mikroorganismen oder Zellwänden der Algen durch ein oder mehrere Verfahren innerhalb der Gruppe bestehend aus Beschallung, Flüssigkeitsscherbruch, Mahlen in der Kugelmühle, Hochdruckpressen, Gefriertauen, Gefrierpressen, enzymatische Digestion, Hydrolysierung und Virusabbau durchgeführt wird.

3. Kontinuierliches Verfahren nach Anspruch 1 oder 2, wobei eine Zellphase aus dem Zweiphasen-Trenner erhalten wird, welche Zellphase eine Trockenheit von mindestens 15 %, bevorzugt mindestens 18 %, am bevorzugtesten mindestens 20 % aufweist.

4. Kontinuierliches Verfahren nach einem der vorhergehenden Ansprüche, wobei die austretende bioorganische Phase eine Trockenheit von mindestens 30 %, bevorzugt eine Trockenheit von mindestens 35 %, am bevorzugtesten eine Trockenheit von mindestens 50 % aufweist.

5. Kontinuierliches Verfahren nach einem der vorhergehenden Ansprüche, wobei eine Schälscheibe die flüssige Phase von dem Zweiphasen-Trenner schält.

6. Kontinuierliches Verfahren nach einem der vorhergehenden Ansprüche, wobei die flüssige Phase und/oder die bioorganische Phase, die die Zellteile enthält, weiter verarbeitet werden, um Zellulose und/oder Stärke zu erhalten, die noch weiter verarbeitet werden könnten, um Alkohol, wie Methanol oder Ethanol oder Alkoholderivate zu erhalten.

7. Verwendung eines Verfahrens nach einem der Ansprüche 1 bis 6 für die Herstellung von Biodiesel oder Biotreibstoffen.

## Revendications

1. Procédé continu pour isoler des huiles à partir d'une suspension de micro-organismes ou d'une suspension d'algue, comprenant la concentration de la suspension de micro-organismes ou de la suspension d'algue dans un séparateur centrifuge à deux phases ayant un empilement de disques séparateurs et fonctionnant sous une force d'au moins 4000 G, de préférence sous une force d'au moins 4500 G, de toute préférence 5000 G, obtenant une phase cellulaire et véhiculant la phase cellulaire hors du séparateur centrifuge à deux phases par une vis sans fin (2);
la libération des huiles par rupture ou perméabilisation des parois cellulaires des cellules dans la phase cellulaire obtenue, obtenant une suspension comprenant les huiles libérées et les parois cellulaires rompues;
la séparation de la suspension comprenant les huiles libérées et les parois cellulaires rompues dans un séparateur centrifuge à trois phases ayant un empilement de disques séparateurs et fonctionnant sous une force d'au moins 4000 G, de préférence sous une force d'au moins 4500 G, de toute préférence 5000 G, obtenant trois phases: une phase huileuse, une phase liquide et une phase bio-organique contenant des parties de cellules, dans lesquelles la phase bio-organique contenant des parties de cellules est véhiculée hors du séparateur centrifuge à trois phases par la vis sans fin (2), et dans laquelle deux disques diviseurs séparent la phase huileuse et la phase liquide, un disque diviseur séparant la phase huileuse et un disque diviseur séparant la phase liquide.

2. Procédé continu selon la revendication 1, dans lequel la libération des huiles par rupture ou perméabilisation des parois cellulaires des micro-organismes ou des parois cellulaires de l'algue est effectuée par un ou plusieurs procédés dans le groupe constitué par sonication, rupture par cisaillement liquide, broyage par billes, pressage sous haute pression, congélation-décongélation, congélation sous pression, digestion enzymatique, hydrolyse, et dégradation par virus.

3. Procédé continu selon la revendication 1 ou 2, dans lequel une phase cellulaire est obtenue à partir du séparateur à deux phases, laquelle phase cellulaire a une sécheresse d'au moins 15%, de préférence d'au moins 18%, de toute préférence d'au moins 20%.

4. Procédé continu selon l'une quelconque des revendications précédentes, dans lequel la phase bio-organique sortante a une sécheresse d'au moins 30%, de préférence une sécheresse d'au moins 35%, de toute préférence une sécheresse d'au moins 50%.

5. Procédé continu selon l'une quelconque des revendications précédentes, dans lequel un disque diviseur sépare la phase liquide hors du séparateur à deux phases.

6. Procédé continue selon l'une quelconque des revendications précédentes, dans lequel la phase liquide et/ou la phase bio-organique contenant les parties de cellules sont plus encore traitées pour obtenir de la cellulose et/ou de l'amidon, qui peuvent ensuite être traités pour obtenir un alcool, comme le méthanol ou l'éthanol, ou des dérivés d'alcool.

7. Utilisation d'un procédé selon l'une quelconque des revendications 1 à 6 pour la production de biodiesel ou de biocombustibles.
